# EUROPEAN PATENT APPLICATION

(11) **EP 4 167 237 A1**
(43) Date of publication of application: **19.04.2023**
(21) Application number: 21202941.7
(22) Date of filing: 15.10.2021
(51) Int. Cl.: G16C 20/10

(54) **METHOD AND SYSTEM FOR RECIPE RECOMMENDATION, STORAGE MEDIUM**

(71) Applicant: Covestro Deutschland AG, 51373 Leverkusen (DE)
(72) Inventor: Zhai, Zack, Pudong, Shanghai, 200126 (CN); Guo, Ruijing, Pudong, Shanghai, 201208 (CN); Gu, Yongming, Pudong New District, Shanghai, 201208 (CN); Sun, Guobin, Pudong, Shanghai, 201204 (CN); Zhu, Erika, Hongkou District,Shanghai, 200434 (CN); Froebel, Sascha, 40591 Düsseldorf (DE); Meyer, Jan, 42329 Wuppertal (DE); Gamez, Jose, 51063 Köln (DE)
(74) Representative: Levpat

(57) **Abstract**

The present invention relates to a method and system for recipe recommendation, and a storage medium. The method comprises: receiving conversion rate-time relationships of empirical recipes, wherein the conversion rate-time relationships are formed jointly according to at least two reaction processes of the empirical recipes that produce a thermosetting resin; constructing molecular dynamic models for the empirical recipes with the conversion rate-time relationships as a target; predicting a material property of the thermosetting resin formed according to the empirical recipes based on the molecular dynamic models, and establishing a mapping with respect to the empirical recipes and the material property; and receiving a target material property of the thermosetting resin, and recommending the recipes for producing the thermosetting resin having the target material property based on the mapping. The method can reduce the experimental scale of the recipes for chemical production.

## Description

### TECHNICAL FIELD

The present application relates to determining recipes of feed materials according to a target material property in the process of producing a thermosetting resin, specifically, to a method for recipe recommendation, a system for recipe recommendation, and a computer-readable storage medium.

### BACKGROUND

Traditionally, the process of determining recipes for the production of a thermosetting resin relies heavily on experimental data. In particular, it may require very large-scale experimentation for the development of a thermosetting resin having a new material property before said resin is put into production. However, massive experiments not only cost lots of resources, but also may delay the development of products.

Therefore, it is imperative to bring forth a solution that can provide recipes prior to experimentation, or at least point out the direction of feasible recipes, so as to reduce the subsequent experimental scale.

### SUMMARY

To address the above issues, the embodiments of the present application provide a method for recipe recommendation, a recipe recommendation system and a computer-readable storage medium utilizing the principle of molecular dynamics, to reduce the experimental scale of determining the recipes for chemical production.

According to one aspect of the present application, a method for recipe recommendation is provided, comprising: receiving conversion rate-time relationships for empirical recipes, wherein the conversion rate-time relationships are formed jointly according to at least two reaction processes of the empirical recipes that produce a thermosetting resin; constructing molecular dynamic models for the empirical recipes with the conversion rate-time relationships as a target; predicting a material property of the thermosetting resin formed according to the empirical recipes based on the molecular dynamic models, and establishing a mapping with respect to the empirical recipes and the material property; and receiving a target material property for the thermosetting resin, and recommending the recipes for producing the thermosetting resin having the target material property based on the mapping.

In some embodiments of the present application, optionally, the at least two reaction processes comprise at least two simultaneous different reaction processes, and the conversion rate-time relationships are established according to the at least two simultaneous different reaction processes; and constructing the molecular dynamic models comprises: constructing molecular dynamic models for the empirical recipes targeting the conversion rate-time relationships of the at least two simultaneous different reaction processes.

In some embodiments of the present application, optionally, the conversion rate-time relationships are conversion rate-time curves, and constructing the molecular dynamic models comprises: identifying a plurality of stages of the at least two simultaneous different reaction processes according to the slopes of the conversion rate-time curves; and performing a molecular dynamics simulation for each stage separately, and thereby forming the molecular dynamic models.

In some embodiments of the present application, optionally, constructing the molecular dynamic models further comprises: receiving adjustments to reaction rates of the at least two simultaneous different reaction processes; and updating the molecular dynamic models according to the adjusted reaction rates for the at least two simultaneous different reaction processes.

In some embodiments of the present application, optionally, constructing the molecular dynamic models further comprises: in the event that the conversion rate of the molecular dynamics simulation reaches saturation before reaching the highest conversion rate of the empirical recipes, then raising the temperature of the molecular dynamics simulation while maintaining the ratio of slopes of the conversion rate-time curves of each stage of the at least two simultaneous different reaction processes, so that the conversion rate of the molecular dynamics simulation reaches the highest conversion rate of the empirical recipes.

In some embodiments of the present application, optionally, constructing the molecular dynamic models further comprises: determining the concentration of each reactant of the empirical recipes; determining the reaction rate constants of the at least two simultaneous different reaction processes separately according to the slopes of their conversion rate-time curves; determining the relative occurrence probability of the at least two simultaneous different reaction processes according to their reaction rate constants; and constructing the molecular dynamic models by taking the concentration of each reactant and the relative occurrence probability of the at least two simultaneous different reaction processes as input parameters.

In some embodiments of the present application, optionally, the at least two simultaneous different reaction processes are an addition reaction process and a radical reaction process that occur at the same time.

In some embodiments of the present application, optionally, the addition reaction process is an addition polymerization process of isocyanate groups and hydroxyl groups, and the radical reaction process is radical polymerization process.

In some embodiments of the present application, optionally, the prediction is made based on the differences among the molecular dynamic models.

In some embodiments of the present application, optionally, establishing the mapping comprises: estimating, in accordance with the differences among the molecular dynamic models, the variation trend of the material property of the thermosetting resin formed according to the empirical recipes; and establishing the mapping in accordance with the empirical recipes, the material property and its variation trend.

In some embodiments of the present application, optionally, the material property can be any one of: material mechanical properties, material flow properties, material dielectric properties and material thermal conductivity.

In some embodiments of the present application, optionally, the recipes comprise components and proportions thereof.

In some embodiments of the present application, optionally, the conversion rate-time relationships are generated according to various curing processes of the empirical recipes, and the method further comprises: recommending a curing process for generating the thermosetting resin having the target material property.

According to another aspect of the present application, a system for recipe recommendation is provided, comprising: a receiving unit configured to receive conversion rate-time relationships for empirical recipes, wherein the conversion rate-time relationships are formed jointly according to at least two reaction processes of the empirical recipes that produce a thermosetting resin; a simulating unit configured to construct molecular dynamic models for the empirical recipes with the conversion rate-time relationships as a target; a mapping unit configured to predict a material property of the thermosetting resin formed according to the empirical recipes based on the molecular dynamic models and to establish a mapping with respect to the empirical recipes and the material property; and a recommending unit configured to receive a target material property for the thermosetting resin, and to recommend the recipes for producing the thermosetting resin having the target material property based on the mapping.

In some embodiments of the present application, optionally, the at least two reaction processes comprise at least two simultaneous different reaction processes, and the conversion rate-time relationships received by the receiving unit are established according to the at least two simultaneous different reaction processes; and the simulating unit is configured to construct molecular dynamic models for the empirical recipes targeting the conversion rate-time relationships of the at least two simultaneous different reaction processes.

In some embodiments of the present application, optionally, the conversion rate-time relationships are conversion rate-time curves, and the simulating unit is configured to: identify a plurality of stages of the at least two simultaneous different reaction processes according to the slopes of the conversion rate-time curves; and perform a molecular dynamics simulation for each stage separately, and thereby form the molecular dynamic models.

In some embodiments of the present application, optionally, the simulating unit is further configured to: receive adjustments to reaction rates of the at least two simultaneous different reaction processes; and update the molecular dynamic models according to the adjusted reaction rates for the at least two simultaneous different reaction processes.

In some embodiments of the present application, optionally, the simulating unit is further configured to: raise the temperature of the molecular dynamics simulation while maintaining the ratio of slopes of the conversion rate-time curves of each stage of the at least two simultaneous different reaction processes, in the event that the conversion rate of the molecular dynamics simulation reaches saturation before reaching the highest conversion rate of the empirical recipes, so that the conversion rate of the molecular dynamics simulation reaches the highest conversion rate of the empirical recipes.

In some embodiments of the present application, optionally, the simulating unit is configured to: determine the concentration of each reactant of the empirical recipes; determine the reaction rate constants of the at least two simultaneous different reaction processes separately according to the slopes of their conversion rate-time curves; determine the relative occurrence probability of the at least two simultaneous different reaction processes according to their reaction rate constants; and construct the molecular dynamic models by taking the concentration of each reactant and the relative occurrence probability of the at least two simultaneous different reaction processes as input parameters.

In some embodiments of the present application, optionally, the at least two simultaneous different reaction processes are an addition reaction process and a radical reaction process that occur at the same time.

In some embodiments of the present application, optionally, the addition reaction process is addition polymerization process of isocyanate groups and hydroxyl groups, and the radical reaction process is radical polymerization process.

In some embodiments of the present application, optionally, the mapping unit is configured to predict the material property based on the differences among the molecular dynamic models.

In some embodiments of the present application, optionally, the mapping unit is configured to: estimate, in accordance with the differences among the molecular dynamic models, the variation trend of the material property of the thermosetting resin formed according to the empirical recipes; and establish the mapping in accordance with the empirical recipes, the material property and its variation trend.

In some embodiments of the present application, optionally, the material property can be any one of: material mechanical properties, material flow properties, material dielectric properties and material thermal conductivity.

In some embodiments of the present application, optionally, the recipes comprise components and proportions thereof.

In some embodiments of the present application, optionally, the conversion rate-time relationships are generated according to various curing processes of the empirical recipes, and the recommending unit is configured to recommend a curing process for generating the thermosetting resin having the target material property based on the molecular dynamic models.

According to yet another aspect of the present application, a computer-readable storage medium is provided, having instructions stored thereon that, when executed by a processor, cause the processor to perform any one of the above methods.

### BRIEF DESCRIPTION OF THE DRAWINGS

The above and other objectives and advantages of the present application will be rendered more complete and clearer from the following detailed description in connection with the drawings. The same or similar element is represented by the same reference numeral.
Fig. 1 illustrates a method for recipe recommendation according to an embodiment of the present application.
Fig. 2 illustrates a system for recipe recommendation according to an embodiment of the present application.
Fig. 3 illustrates the working principle of molecular dynamics according to an embodiment of the present application.
Fig. 4 illustrates the working principle of molecular dynamics according to an embodiment of the present application.

### DETAILED DESCRIPTION OF THE INVENTION

For brevity and illustrative purposes, this document mainly refers to its exemplary embodiments to describe the principles of the present application. Persons skilled in the art, however, would easily recognize that the same principles can be equally applied to all types of the recipe recommendation method, recipe recommendation system and computer-readable storage medium, and these same or similar principles can be implemented therein. Any such change would not deviate from the true spirit and scope of the present application.

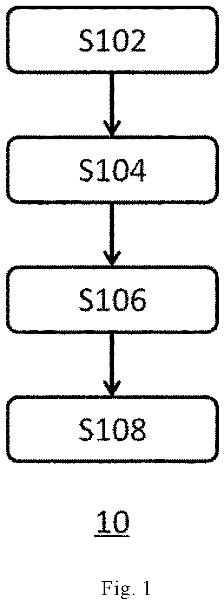

According to one aspect of the present application, a method for recipe recommendation is provided. As illustrated in Fig. 1, the method for recipe recommendation 10 comprises the following steps: in step S102, receiving conversion rate-time relationships for empirical recipes; in step S104, constructing molecular dynamic models for the empirical recipes with the conversion rate-time relationships as a target; in step S106, predicting a material property of the thermosetting resin formed according to the empirical recipes based on the molecular dynamic models, and establishing a mapping with respect to the empirical recipes and the material property; and in step S108, receiving a target material property for the thermosetting resin, and recommending the recipes for producing the thermosetting resin having the target material property based on the mapping. In some embodiments of the present application, the recipes comprise the components and proportions thereof for producing a thermosetting resin.

The method for recipe recommendation 10, utilizing the principle of molecular dynamics, establishes a connection between the empirical recipes and the material property of the product formed therewith, and further applies such connection to recommend recipes for producing a thermosetting resin having the target material property. Those skilled in the art, in view of the recommended recipes, can test and verify through experiments whether the resulting thermosetting resin product achieves the target material property, and can further adopt or fine-tune the recommended recipes according to the experimental results, thereby determining the recipes to be put into production. As such, the method for recipe recommendation in some examples of the present invention can significantly reduce the experimental scale, while at the same time save time for research and development, and investment in feed materials. The working principle of the method for recipe recommendation 10 will be described in detail hereinafter.

In the method for recipe recommendation 10, the step S102 involves receiving conversion rate-time relationships for empirical recipes, wherein the conversion rate-time relationships are formed jointly according to at least two reaction processes of the empirical recipes that produce a thermosetting resin (the detailed reactions can be found in CN104974502B, the whole content of which is incorporated into this document by way of reference). Such relationships can reflect the change of the conversion rate of the product over time. Generally speaking, the conversion rate-time relationships correspond to the empirical recipes. Therefore, the product property obtained by computer simulation using the conversion rate-time relationships would also be equivalent to that obtained by actually engaging the empirical recipes in reaction.

The so-called empirical recipes refer to those purposefully recording the process of producing a thermosetting resin putting said recipes into production. For example, the empirical recipes can be derived from data of simulated production in the laboratory, or historical data of practical production. Depending on the data from different sources, the empirical recipes may have different components, and may also have different proportions thereof even if the components are the same. The recipes based on different components and proportions thereof can always form the molecular dynamic models described in detail hereinafter. In other words, the molecular dynamic models correspond to the components and proportions thereof. Since the conversion rate-time relationships for the empirical recipes may include the conversion of each recipe in the production process over time, the conversion rate-time relationships can serve as the basis for molecular dynamics to simulate the reaction processes.

In the method for recipe recommendation 10, the step S 104 involves constructing molecular dynamic models for the empirical recipes with the conversion rate-time relationships as a target. Molecular dynamics is a set of molecular simulation methods which mainly rely on computers to simulate motions of the molecular and atomic systems, and it is a multi-body simulation method. By simulating the state of motion of molecules and atoms in a certain period of time, one can observe the evolution of the system over time from a dynamic point of view. In step S104, the conversion rate-time relationships for empirical recipes received in step S102 serve as input parameters for simulating molecular dynamic models, and further the models of the reaction processes are built up using molecular dynamics.

As stated above, the molecular dynamic models correspond to the components and proportions thereof. Thus, the built-up molecular dynamic models can be used to study various properties of the thermosetting resin produced according to specific components and proportions thereof, such as a material property. This makes it possible to study the reasons why the components and proportions of feed materials affect the material property of the final product from the molecular level, and the results of this study can in turn affect the conscious selection of the empirical recipes. For example, the variation trend of the feed materials relative to the corresponding material property in the results of this study can be used to guide empirical recipes for experimentation, and the data of conversion rate-time relationships in the experiments can also serve as input parameters for simulating molecular dynamic models.

In some embodiments of the present application, the reaction process can be divided into multiple reactions for study, so as to construct precise molecular dynamic models. In some examples, the divided reactions do not necessarily vary according to different empirical recipes. For instance, when different components and proportions thereof are involved in reaction, the type of reaction for generating a thermosetting resin remains the same. In some examples, the reaction process for generating a thermosetting resin comprises at least two simultaneous different reaction processes. In a more specific example, the at least two simultaneous different reaction processes may be an addition reaction process (forming carbon chains) and a radical reaction process (forming crosslinks). More specifically, the addition reaction process may be addition polymerization process of isocyanate groups and hydroxyl groups, and the radical reaction process may be radical polymerization process. Under this premise, the conversion rate-time relationships received in step S102 may be established according to at least two simultaneous different reaction processes. In a more specific example, the conversion rate-time relationships may be established separately according to the addition reaction process and the radical reaction process. Furthermore, in the process of constructing molecular dynamic models in step S104, molecular dynamic models for the empirical recipes are constructed targeting the conversion rate-time relationships of the at least two simultaneous different reaction processes (in a more specific example, targeting the conversion rate-time relationship of the addition reaction process, and the conversion rate-time relationship of the radical reaction process). Dividing the reaction process would help simulate the reaction process in a more realistic manner, so that the generated models can better reflect the actual situation, and further precisely predict the material property of the thermosetting resin.

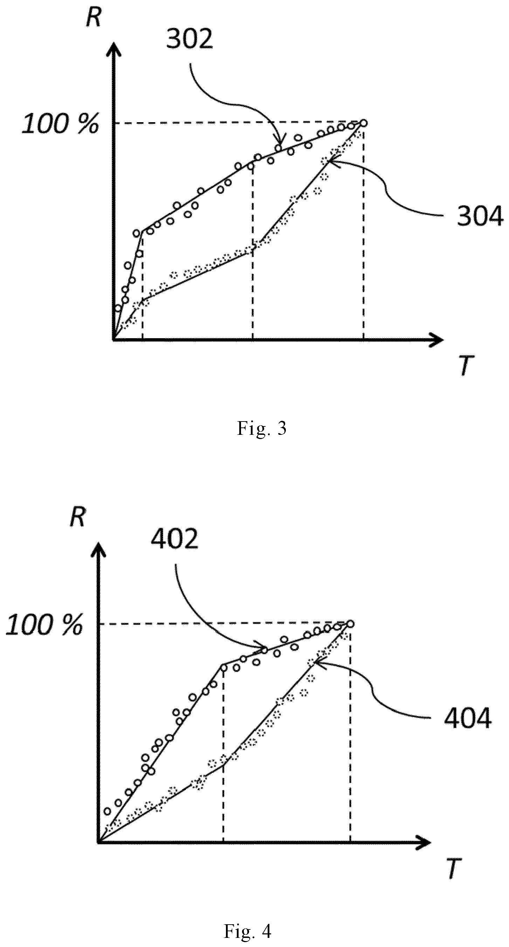

In some embodiments of the present application, for the convenience of studying the conversion rate-time relationships, the conversion rate-time relationships may be expressed as conversion rate-time curves. Moreover, in the process of constructing molecular dynamic models in step S104, the conversion rate-time curves can be divided into multiple stages for study. In some examples, in step S104, multiple stages of at least two simultaneous different reaction processes can be identified according to the slopes of the conversion rate-time curves. For instance, the processes having different slopes are identified as different stages. Fig. 3 illustrates the conversion rate-time curve 302 of the addition reaction process and the conversion rate-time curve 304 of the radical reaction process, wherein the lateral axis T represents time, and the vertical axis R represents the conversion rate. The conversion rate-time curves 302 and 304 can be obtained by such as linear regression fitting based on the data extracted from the reaction processes. For example, the origins in the figure represent the data extracted from the reaction processes, and they can be fitted to obtain the conversion rate-time curves 302 and 304.

According to the slopes of the conversion rate-time curve 302 of the addition reaction process and the conversion rate-time curve 304 of the radical reaction process, the addition reaction process and the radical reaction process can be correspondingly divided into 3 stages (from left to right, named as the first stage, the second stage and the third stage respectively).

Similarly, in the embodiment shown in Fig. 4, according to the slopes of the conversion rate-time curve 402 of the addition reaction process and the conversion rate-time curve 404 of the radical reaction process, the addition reaction process and the radical reaction process can be correspondingly divided into 2 stages (from left to right, named as the first stage and the second stage respectively). In other examples, according to the slopes of the conversion rate-time curves, the reaction processes may also be divided into other number of stages.

Each stage of the two reaction processes shown in Figs. 3 and 4 corresponds to each other. In other words, when the addition reaction is in the first stage, the radical reaction is also in the first stage. However, in other examples, each stage of the two reaction processes may also be irrelevant.

In step S104, molecular dynamics simulation can be performed separately for each stage to form molecular dynamic models. Dividing the reaction process into multiple stages for study would help simulate the reaction process in a more realistic manner, so that the generated models can better reflect the actual situation, and further precisely predict the material property of the thermosetting resin.

In some examples, the step S104 may also comprise receiving adjustments to reaction rates of the at least two simultaneous different reaction processes; and updating the molecular dynamic models according to the adjusted reaction rates for the at least two simultaneous different reaction processes. In some cases, the reaction rates of the simulation processes can be fine-tuned to finally make simulated conversion rate curves match with experiments, thereby improving the precision of the constructed dynamic models.

In some examples, given that the final results of constructing molecular dynamic models merely on the basis of the conversion rate-time relationships may not meet expectations, for example, the conversion rate of the molecular dynamics simulation fails to reach the highest conversion rate of the empirical recipes, the process of constructing the molecular dynamic models in step S104 can further comprise the following steps: in the event that the conversion rate of the molecular dynamics simulation reaches saturation before reaching the highest conversion rate of the empirical recipes, then raising the temperature of the molecular dynamics simulation while maintaining the ratio of slopes of the conversion rate-time curves of each stage of the at least two simultaneous different reaction processes, so that the conversion rate of the molecular dynamics simulation reaches the highest conversion rate of the empirical recipes. This process will ensure that the constructed molecular dynamic models reach the highest conversion rate of the empirical recipes, thereby being more in line with the actual situation.

In some examples, the process of constructing the molecular dynamic models in step S104 takes into account factors such as the concentration of each reactant and the reaction rate constants of the reaction process. Specifically, this can be achieved by the following steps: determining the concentration of each reactant of the empirical recipes; determining the reaction rate constants of the at least two simultaneous different reaction processes separately according to the slopes of their conversion rate-time curves; determining the relative occurrence probability of the at least two simultaneous different reaction processes according to their reaction rate constants; and constructing the molecular dynamic models by taking the concentration of each reactant and the relative occurrence probability of the at least two simultaneous different reaction processes as input parameters.

In the method for recipe recommendation 10, the step S106 involves predicting a material property of the thermosetting resin formed according to the empirical recipes based on the molecular dynamic models, and establishing a mapping with respect to the empirical recipes and the material property. In step S106, first, it is necessary to use the molecular dynamic models formed in step S104 to predict the material property of the thermosetting resin formed according to its corresponding recipes. As such, the material property can be predicted from the microstructural level. On the one hand, it can save a lot of repeated experiments, on the one hand, it can also study the influence of microstructure on the material property.

The step S104 as stated above actually establishes a connection between the recipes and the microstructure of the product at the molecular level, and the step S 106 further establishes a connection between the microstructure of the product and various performance indices of the product, which makes it possible to establish a connection between the empirical recipes and the material property. The step S106 also establishes a mapping with respect to the empirical recipes and the material property. The "mapping" in the context of the present application refers to the one that can reflect the connection between the empirical recipes and the material property of the thermosetting resin formed on the basis of said recipes.

In some examples, such mapping can be expressed as a mapping of several discrete values. For instance, the mapping comprises a mapping of some recipes to some material properties. These recipes may comprise some empirical recipes (such as all of the empirical recipes involved in step S104). The mapping in this case comprises a mapping of these empirical recipes to the material properties predicted according to step S106.

In some embodiments of the present application, the prediction is made based on the differences among the molecular dynamic models. The molecular dynamic models can be used to predict material properties of the thermosetting resin formed according to empirical recipes. Moreover, the differences among the molecular dynamic models can also be used to assess differences among the material properties of the thermosetting resin. For instance, the simulation results of the molecular dynamic models show that when the empirical recipe X gets involved in reaction, the final thermosetting resin would comprise 40% of product I and 60% of product II; when the empirical recipe Y gets involved in reaction, the final thermosetting resin would comprise 50% of product I and 50% of product II. The product I has better tensile properties than the product II. Therefore, it is possible to at least predict, in view of the differences between these two models, that the thermosetting resin obtained by engaging the empirical recipe Y in reaction would have better tensile properties.

In some embodiments of the present application, the process of establishing a mapping in step S106 comprises: estimating, in accordance with the differences among the molecular dynamic models, the variation trend of the material property of the thermosetting resin formed according to the empirical recipes; and establishing the mapping in accordance with the empirical recipes, the material property and its variation trend. The following table illustrates a mapping. In this table, the empirical blending ratios of components A+B involved in reaction are blending ratio 1, blending ratio 2 and blending ratio 3, and it can be estimated according to the molecular dynamic models that the material properties thereof are material property 1, material property 2 and material property 3; the empirical blending ratios of components A+B+C involved in reaction are blending ratio 4, blending ratio 5 and blending ratio 6, and it can be estimated according to the molecular dynamic models that the material properties thereof are material property 4, material property 5 and material property 6.

Since the examples of empirical recipes (consisting of empirical components and corresponding empirical blending ratios) cannot be exhausted, it is possible to estimate, based on the differences among the molecular dynamic models corresponding to various recipes, the variation trend of the material property of the generated thermosetting resin. For instance, when the empirical components A+B get involved in reaction, if the blending ratio 1 is 50% A and 50% B, the final thermosetting resin would comprise 40% product I and 60% product II; if the blending ratio 2 is 40% A and 60% B, the final thermosetting resin would comprise 50% product I and 50% product II; if the blending ratio 3 is 30% A and 70% B, the final thermosetting resin would comprise 60% product I and 40% product II. The product I has better material property (for example, mechanical properties (more specifically, tensile properties), material flow properties, material dielectric properties, material thermal conductivity, etc.) than the product II. Therefore, it can be predicted at least on the basis of the above differences that with the decrease of the proportion of A and the increase of the proportion of B, the material property of the generated thermosetting resin would be gradually improved.

Although *f* and *g* in the following table cannot correspond to specific empirical values, at least the underlying trend is obvious. The *f* and *g* included in the generated mapping would indicate such a trend. Based on this mapping, if the blending ratio is 45% A and 55% B, the material property of the final thermosetting resin should fall between the material property 1 and the material property 2. In the following table, *h* and *i* can also record the variation trend of the material property corresponding to the components and proportions thereof. As such, the generated mapping can reflect the empirical recipes, the material property and its variation trend. Such mapping can be used for guidance on recipes for the production of a thermosetting resin having the target material property.

| | | |
|---|---|---|
| Components: A+B | Blending ratio 1 | Material property 1 |
| | *f* | |
| | Blending ratio 2 | Material property 2 |
| | *g* | |
| | Blending ratio 3 | Material property 3 |
| Components: A+B+C | Blending ratio 4 | Material property 4 |
| | *h* | |
| | Blending ratio 5 | Material property 5 |
| | *i* | |
| | Blending ratio 6 | Material property 6 |

It should be noted that the present invention does not limit the form of existence of the mapping, so long as a connection between the recipes and the material property of the thermosetting resin can be established according to said mapping. In some examples, the mapping may exist in the form of a computer readable lookup table.

In the method for recipe recommendation 10, the step S108 involves receiving a target material property for the thermosetting resin, and recommending the recipes for producing the thermosetting resin having the target material property based on the mapping. After the mapping is obtained, the recipes for reaction can be recommended according to the expected target material property. Researchers can decompose the recommended recipes for experimental verification. Since the method for recipe recommendation 10 at least gives the direction of recipes through a molecular dynamics method, it is possible to significantly reduce the experimental scale, while at the same time save time for research and development, and investment in feed materials.

In some examples, the conversion rate-time relationships can be generated according to various curing processes of the empirical recipes, and the method for recipe commendation 10 further comprises: recommending a curing process for generating the thermosetting resin having the target material property (not shown in Fig. 1). This process helps to recommend one or more feasible curing processes, and can generate the thermosetting resin having the target material property based on the recommended curing processes.

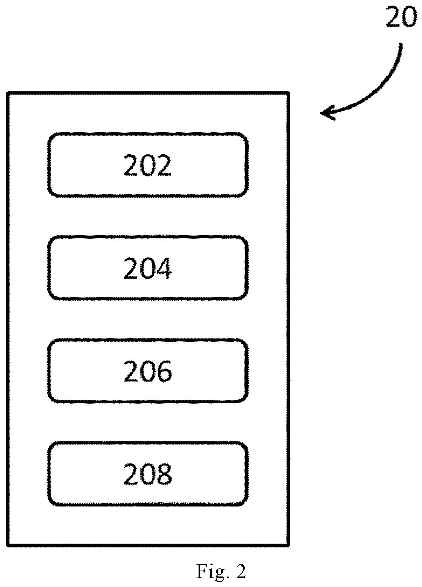

According to another aspect of the present application, a system for recipe recommendation is provided. As illustrated in Fig. 2, the system for recipe recommendation 20 comprises a receiving unit 202, a simulating unit 204, a mapping unit 206, and a recommending unit 208. The system for recipe recommendation 20, utilizing the principle of molecular dynamics, establishes a connection between the empirical recipes and the material property of the product formed therewith, and further applies such connection to recommend recipes for producing the thermosetting resin having the target material property. Those skilled in the art, in view of the recommended recipes, can test and verify through experiments whether the resulting thermosetting resin product achieves the target material property, and can further adopt or fine-tune the recommended recipes according to the experimental results, thereby determining the recipes to be put into production. As such, the system for recipe recommendation according to some embodiments of the present invention can significantly reduce the experimental scale, while at the same time save time for research and development, and investment in feed materials. The working principle of the system for recipe recommendation 20 will be described in detail hereinafter.

The receiving unit 202 of the system for recipe recommendation 20 is configured to receive conversion rate-time relationships for empirical recipes, wherein the conversion rate-time relationships are formed jointly according to at least two reaction processes of the empirical recipes that produce a thermosetting resin. Such relationships can reflect the change of the conversion rate of the product over time. Generally speaking, the conversion rate-time relationships correspond to the empirical recipes. Therefore, the product property obtained by computer simulation using the conversion rate-time relationships would also be equivalent to that obtained by actually engaging the empirical recipes in reaction.

The so-called empirical recipes refer to those purposefully recording the process of producing a thermosetting resin putting said recipes into production. For example, the empirical recipes can be derived from data of simulated production in the laboratory, or historical data of practical production. Depending on the data from different sources, the empirical recipes may have different components, and may also have different proportions thereof even if the components are the same. The recipes based on different components and proportions thereof can always form the molecular dynamic models described in detail hereinafter. In other words, the molecular dynamic models correspond to the components and proportions thereof. Since the conversion rate-time relationships for the empirical recipes may include the conversion of each recipe in the production process over time, the conversion rate-time relationships can serve as the basis for molecular dynamics to simulate the reaction processes.

The simulating unit 204 of the system for recipe recommendation 20 is configured to construct molecular dynamic models for the empirical recipes with the conversion rate-time relationships as a target. Molecular dynamics is a set of molecular simulation methods which mainly rely on computers to simulate motions of the molecular and atomic systems, and it is a multi-body simulation method. By simulating the state of motion of molecules and atoms in a certain period of time, one can observe the evolution of the system over time from a dynamic point of view. The simulating unit 204 takes the conversion rate-time relationships for empirical recipes received by the receiving unit 202 as input parameters for simulating molecular dynamic models, and then builds up models of the reaction processes using molecular dynamics.

As stated above, the molecular dynamic models correspond to the components and proportions thereof. Thus, the built-up molecular dynamic models can be used to study various properties of the thermosetting resin produced according to specific components and proportions thereof, such as a material property. This makes it possible to study the reasons why the components and proportions of feed materials affect the material property of the final product from the molecular level, and the results of this study can in turn affect the conscious selection of the empirical recipes. For example, the variation trend of the feed materials relative to the corresponding material property in the results of this study can be used to guide empirical recipes for experimentation, and the data of conversion rate-time relationships in the experiments can also serve as input parameters for simulating molecular dynamic models.

In some embodiments of the present application, the reaction process can be divided into multiple reactions for study, so as to construct precise molecular dynamic models. In some examples, the divided reactions do not necessarily vary according to different empirical recipes. For instance, when different components and proportions thereof are involved in reaction, the type of reaction for generating a thermosetting resin remains the same. In some examples, the reaction process for generating a thermosetting resin comprises at least two simultaneous different reaction processes. In a more specific example, the at least two simultaneous different reaction processes may be an addition reaction process (forming carbon chains) and a radical reaction process (forming crosslinks). Under this premise, the conversion rate-time relationships received by the receiving unit 202 may be established according to at least two simultaneous different reaction processes. In a more specific example, the conversion rate-time relationships may be established separately according to the addition reaction process and the radical reaction process. More specifically, the addition reaction process may be addition polymerization process of isocyanate groups and hydroxyl groups, and the radical reaction process may be radical polymerization process. Furthermore, the simulating unit 204 can be configured to construct molecular dynamic models for the empirical recipes targeting the conversion rate-time relationships of the at least two simultaneous different reaction processes (in a more specific example, targeting the conversion rate-time relationship of the addition reaction process, and the conversion rate-time relationship of the radical reaction process). Dividing the reaction process would help simulate the reaction process in a more realistic manner, so that the generated models can better reflect the actual situation, and further precisely predict the material property of the thermosetting resin.

In some embodiments of the present application, for the convenience of studying the conversion rate-time relationships, the conversion rate-time relationships may be expressed as conversion rate-time curves. Moreover, the simulating unit 204 can divide the conversion rate-time curves into multiple stages for study. In some examples, the simulating unit 204 can identify a plurality of stages of the at least two simultaneous different reaction processes according to the slopes of the conversion rate-time curves, for example, identify the processes with different slopes as different stages. Fig. 3 illustrates the conversion rate-time curve 302 of the addition reaction process and the conversion rate-time curve 304 of the radical reaction process, wherein the lateral axis T represents time, and the vertical axis R represents the conversion rate. The conversion rate-time curves 302 and 304 can be obtained by such as linear regression fitting based on the data extracted from the reaction processes. For example, the origins in the figure represent the data extracted from the reaction processes, and they can be fitted to obtain the conversion rate-time curves 302 and 304.

According to the slopes of the conversion rate-time curve 302 of the addition reaction process and the conversion rate-time curve 304 of the radical reaction process, the addition reaction process and the radical reaction process can be correspondingly divided into 3 stages (from left to right, named as the first stage, the second stage and the third stage respectively).

Similarly, in the embodiment shown in Fig. 4, according to the slopes of the conversion rate-time curve 402 of the addition reaction process and the conversion rate-time curve 404 of the radical reaction process, the addition reaction process and the radical reaction process can be correspondingly divided into 2 stages (from left to right, named as the first stage and the second stage respectively). In other examples, according to the slopes of the conversion rate-time curves, the reaction processes may also be divided into other number of stages.

Each stage of the two reaction processes shown in Figs. 3 and 4 corresponds to each other. In other words, when the addition reaction is in the first stage, the radical reaction is also in the first stage. However, in other examples, each stage of the two reaction processes may also be irrelevant.

The simulating unit 204 can perform molecular dynamics simulation separately for each stage to form molecular dynamic models. Dividing the reaction process into multiple stages for study would help simulate the reaction process in a more realistic manner, so that the generated models can better reflect the actual situation, and further precisely predict the material property of the thermosetting resin.

In some examples, the simulating unit 204 of the system for recipe recommendation 20 can also receive adjustments to reaction rates of the at least two simultaneous different reaction processes; and update the molecular dynamic models according to the adjusted reaction rates for the at least two simultaneous different reaction processes. In some cases, the reaction rates of the simulation processes can be fine-tuned to finally make simulated conversion rate curves match with experiments, thereby improving the precision of the constructed dynamic models.

In some examples, given that the final results of constructing molecular dynamic models merely on the basis of the conversion rate-time relationships may not meet expectations, for example, the conversion rate of the molecular dynamics simulation fails to reach the highest conversion rate of the empirical recipes, the simulating unit 204 of the system for recipe recommendation 20 can also be configured to: raise the temperature of the molecular dynamics simulation while maintaining the ratio of slopes of the conversion rate-time curves of each stage of the at least two simultaneous different reaction processes, in the event that the conversion rate of the molecular dynamics simulation reaches saturation before reaching the highest conversion rate of the empirical recipes, so that the conversion rate of the molecular dynamics simulation reaches the highest conversion rate of the empirical recipes. This process will ensure that the constructed molecular dynamic models reach the highest conversion rate of the empirical recipes, thereby being more in line with the actual situation.

In some examples, the process of the simulating unit 204 constructing the molecular dynamic models takes into account factors such as the concentration of each reactant and the reaction rate constants of the reaction process. Specifically, the simulating unit 204 can: determine the concentration of each reactant of the empirical recipes; determine the reaction rate constants of the at least two simultaneous different reaction processes separately according to the slopes of their conversion rate-time curves; determine the relative occurrence probability of the at least two simultaneous different reaction processes according to their reaction rate constants; and construct the molecular dynamic models by taking the concentration of each reactant and the relative occurrence probability of the at least two simultaneous different reaction processes as input parameters.

The mapping unit 206 of the system for recipe recommendation 20 is configured to predict a material property of the thermosetting resin formed according to the empirical recipes based on the molecular dynamic models, and establish a mapping with respect to the empirical recipes and the material property. The mapping unit 206 first needs to utilize the molecular dynamic models formed by the simulating unit 204 to predict the material property of the thermosetting resin formed according to its corresponding recipes. As such, the material property can be predicted from the microstructural level. On the one hand, it can save a lot of repeated experiments, on the one hand, it can also study the influence of microstructure on the material property.

The simulating unit 204 actually establishes a connection between the recipes and the microstructure of the product at the molecular level, and the mapping unit 206 further establishes a connection between the microstructure of the product and various performance indices of the product, which makes it possible to establish a connection between the empirical recipes and the material property. The mapping unit 206 also establishes a mapping with respect to the empirical recipes and the material property. The "mapping" in the context of the present application refers to the one that can reflect the connection between the empirical recipes and the material property of the thermosetting resin formed on the basis of said recipes.

In some examples, such mapping can be expressed as a mapping of several discrete values. For instance, the mapping comprises a mapping of some recipes to some material properties. These recipes may comprise some empirical recipes. In this case, the mapping comprises a mapping of these empirical recipes to the material properties predicted according to the mapping unit 206.

In some embodiments of the present application, the mapping unit 206 predicts the material property based on the differences among the molecular dynamic models. The molecular dynamic models can be used to predict material properties of the thermosetting resin formed according to empirical recipes. Moreover, the differences among the molecular dynamic models can also be used to assess differences among the material properties of the thermosetting resin. For instance, the simulation results of the molecular dynamic models show that when the empirical recipe X gets involved in reaction, the final thermosetting resin would comprise 40% of product I and 60% of product II; when the empirical recipe Y gets involved in reaction, the final thermosetting resin would comprise 50% of product I and 50% of product II. The product I has better tensile properties than the product II. Therefore, it is possible to at least predict, in view of the differences between these two models, that the thermosetting resin obtained by engaging the empirical recipe Y in reaction would have better tensile properties.

In some embodiments of the present application, the mapping unit 206 is configured to: estimate, in accordance with the differences among the molecular dynamic models, the variation trend of the material property of the thermosetting resin formed according to the empirical recipes; and establish the mapping in accordance with the empirical recipes, the material property and its variation trend. The following table illustrates a mapping. In this table, the empirical blending ratios of components A+B involved in reaction are blending ratio 1, blending ratio 2 and blending ratio 3, and it can be estimated according to the molecular dynamic models that the material properties thereof are material property 1, material property 2 and material property 3. The empirical blending ratios of components A+B+C involved in reaction are blending ratio 4, blending ratio 5 and blending ratio 6, and it can be estimated according to the molecular dynamic models that the material properties thereof are material property 4, material property 5 and material property 6.

Since the examples of empirical recipes (consisting of empirical components and corresponding empirical blending ratios) cannot be exhausted, it is possible to estimate, based on the differences among the molecular dynamic models corresponding to various recipes, the variation trend of the material property of the generated thermosetting resin. For instance, when the empirical components A+B get involved in reaction, if the blending ratio 1 is 50% A and 50% B, the final thermosetting resin would comprise 40% product I and 60% product II; if the blending ratio 2 is 40% A and 60% B, the final thermosetting resin would comprise 50% product I and 50% product II; if the blending ratio 3 is 30% A and 70% B, the final thermosetting resin would comprise 60% product I and 40% product II. The product I has better material property (for example, mechanical properties (more specifically, tensile properties), material flow properties, material dielectric properties, material thermal conductivity, etc.) than the product II. Therefore, it can be predicted at least on the basis of the above differences that with the decrease of the proportion of A and the increase of the proportion of B, the material property of the generated thermosetting resin would be gradually improved.

Although *f* and *g* in the following table cannot correspond to specific empirical values, at least the underlying trend is obvious. The *f* and *g* included in the generated mapping would indicate such a trend. Based on this mapping, if the blending ratio is 45% A and 55% B, the material property of the final thermosetting resin would fall between the material property 1 and the material property 2. In the following table, *h* and *i* can also record the variation trend of the material property corresponding to the components and proportions thereof. As such, the generated mapping can reflect the empirical recipes, the material property and its variation trend. Such mapping can be used for guidance on recipes for the production of a thermosetting resin having the target material property.

| | | |
|---|---|---|
| Components: A+B | Blending ratio 1 | Material property 1 |
| | *f* | |
| | Blending ratio 2 | Material property 2 |
| | *g* | |
| | Blending ratio 3 | Material property 3 |
| Components: A+B+C | Blending ratio 4 | Material property 4 |
| | *h* | |
| | Blending ratio 5 | Material property 5 |
| | *i* | |
| | Blending ratio 6 | Material property 6 |

It should be noted that the present invention does not limit the form of existence of the mapping, so long as a connection between the recipes and the material property of the thermosetting resin can be established according to said mapping. In some examples, the mapping may exist in the form of a computer readable lookup table.

The recommending unit 208 of the system for recipe recommendation 20 is configured to receive a target material property for the thermosetting resin, and recommend the recipes for producing the thermosetting resin having the target material property based on the mapping. After the mapping is obtained, the recipes for reaction can be recommended according to the expected target material property. Researchers can decompose the recommended recipes for experimental verification. Since the system for recipe recommendation 20 at least gives the direction of recipes through a molecular dynamics method, it is possible to significantly reduce the experimental scale, while at the same time save time for research and development, and investment in feed materials.

In some examples, the conversion rate-time relationships can be generated according to various curing processes of the empirical recipes, and the recommending unit 208 is further configured to recommend a curing process for generating the thermosetting resin having the target material property. This process helps to recommend one or more feasible curing processes, and can generate the thermosetting resin having the target material property based on these recommended curing processes. According to yet another aspect of the present application, a computer-readable storage medium is provided, having instructions stored thereon that, when executed by a processor, cause the processor to perform any one of the above methods. The computer-readable storage medium as mentioned in the present application includes various types of computer storage media, and may be any available medium that can be accessed by a general-purpose or special-purpose computer. For example, the computer-readable medium may include RAM, ROM, EPROM, E²PROM, registers, hard disks, removable disks, CD-ROM or other optical disc storage, magnetic disk storage or other magnetic storage devices, or any other transitory or non-transitory medium that can be used to carry or store expected program code units in the form of instructions or data structures and can be accessed by a general-purpose or special-purpose computer or a general-purpose or special-purpose processor. The disk used herein refers to the means to copy data magnetically, whereas the disc refers to the means to copy data optically with laser. The combination of the above media should be included in the protection scope of the computer-readable medium. An exemplary storage medium is coupled to the processor so that the processor can read and write information from/to the storage medium. In an alternative solution, the storage medium may be integrated into the processor. Both the processor and the storage medium may reside in ASIC. The ASIC may reside in the user terminal. In an alternative solution, the processor and the storage medium may reside as discrete components in the user terminal.

The above are just embodiments of the present application, but the protection scope of the present application is not limited thereto. Those skilled in the art, in view of the technical scope disclosed in the present application, can conceive of other feasible changes or substitutions which all fall within the protection scope of the present application. Where there is no conflict, the embodiments of the present application and the features in the embodiments may also be combined with each other. The protection scope of the present application is limited by the attached claims.

## Claims

1. A method for recipe recommendation, comprising:
receiving conversion rate-time relationships for empirical recipes, wherein the conversion rate-time relationships are formed jointly according to at least two reaction processes of the empirical recipes that produce a thermosetting resin;
constructing molecular dynamic models for the empirical recipes with the conversion rate-time relationships as a target;
predicting a material property of the thermosetting resin formed according to the empirical recipes based on the molecular dynamic models, and establishing a mapping with respect to the empirical recipes and the material property; and
receiving a target material property for the thermosetting resin, and recommending the recipes for producing the thermosetting resin having the target material property based on the mapping.

2. The method of claim 1, wherein the at least two reaction processes comprise at least two simultaneous different reaction processes, and the conversion rate-time relationships are established according to the at least two simultaneous different reaction processes; and
constructing the molecular dynamic models comprises: constructing molecular dynamic models for the empirical recipes targeting the conversion rate-time relationships of the at least two simultaneous different reaction processes.

3. The method of claim 1 or 2, wherein the conversion rate-time relationships are conversion rate-time curves, and constructing the molecular dynamic models comprises:
identifying a plurality of stages of the at least two simultaneous different reaction processes according to the slopes of the conversion rate-time curves; and
performing a molecular dynamics simulation for each stage separately, and thereby forming the molecular dynamic models.

4. The method of any of claims 1-3, wherein constructing the molecular dynamic models further comprises:
in the event that the conversion rate of the molecular dynamics simulation reaches saturation before reaching the highest conversion rate of the empirical recipes, then raising the temperature of the molecular dynamics simulation while maintaining the ratio of slopes of the conversion rate-time curves of each stage of the at least two simultaneous different reaction processes, so that the conversion rate of the molecular dynamics simulation reaches the highest conversion rate of the empirical recipes.

5. The method of any of claims 1-4, wherein constructing the molecular dynamic models further comprises:
determining the concentration of each reactant of the empirical recipes;
determining the reaction rate constants of the at least two simultaneous different reaction processes separately according to the slopes of their conversion rate-time curves;
determining the relative occurrence probability of the at least two simultaneous different reaction processes according to their reaction rate constants; and
constructing the molecular dynamic models by taking the concentration of each reactant and the relative occurrence probability of the at least two simultaneous different reaction processes as input parameters.

6. The method of any of claims 1-5, wherein the at least two simultaneous different reaction processes are an addition reaction process and a radical reaction process that occur at the same time.

7. The method of any of claims 1-6, wherein the addition reaction process is addition polymerization process of isocyanate groups and hydroxyl groups, and the radical reaction process is radical polymerization process.

8. The method of any of claims 1-7, wherein establishing the mapping comprises:
estimating, in accordance with the differences among the molecular dynamic models, the variation trend of the material property of the thermosetting resin formed according to the empirical recipes; and
establishing the mapping in accordance with the empirical recipes, the material property and its variation trend.

9. A system for recipe recommendation, comprising:
a receiving unit configured to receive conversion rate-time relationships for empirical recipes, wherein the conversion rate-time relationships are formed jointly according to at least two reaction processes of the empirical recipes that produce a thermosetting resin;
a simulating unit configured to construct molecular dynamic models for the empirical recipes with the conversion rate-time relationships as a target;
a mapping unit configured to predict a material property of the thermosetting resin formed according to the empirical recipes based on the molecular dynamic models and to establish a mapping with respect to the empirical recipes and the material property; and
a recommending unit configured to receive a target material property for the thermosetting resin, and to recommend the recipes for producing the thermosetting resin having the target material property based on the mapping.

10. The system of claim 9, wherein the at least two reaction processes comprise at least two simultaneous different reaction processes, and the conversion rate-time relationships received by the receiving unit are established according to the at least two simultaneous different reaction processes; and
the simulating unit configured to construct molecular dynamic models for the empirical recipes targeting the conversion rate-time relationships of the at least two simultaneous different reaction processes.

11. The system of claim 9 or 10, wherein the conversion rate-time relationships are conversion rate-time curves, and the simulating unit is configured to:
identify a plurality of stages of the at least two simultaneous different reaction processes according to the slopes of the conversion rate-time curves; and
perform a molecular dynamics simulation for each stage separately, and thereby form the molecular dynamic models.

12. The system of any of claims 9-11, wherein the simulating unit is further configured to:
raise the temperature of the molecular dynamics simulation while maintaining the ratio of slopes of the conversion rate-time curves of each stage of the at least two simultaneous different reaction processes, in the event that the conversion rate of the molecular dynamics simulation reaches saturation before reaching the highest conversion rate of the empirical recipes, so that the conversion rate of the molecular dynamics simulation reaches the highest conversion rate of the empirical recipes.

13. The system of any of claims 9-12, wherein the simulating unit is configured to:
determine the concentration of each reactant of the empirical recipes;
determine the reaction rate constants of the at least two simultaneous different reaction processes separately according to the slopes of their conversion rate-time curves;
determine the relative occurrence probability of the at least two simultaneous different reaction processes according to their reaction rate constants; and
construct the molecular dynamic models by taking the concentration of each reactant and the relative occurrence probability of the at least two simultaneous different reaction processes as input parameters.

14. The system of any of claims 9-13, wherein the mapping unit is configured to:
estimate, in accordance with the differences among the molecular dynamic models, the variation trend of the material property of the thermosetting resin formed according to the empirical recipes; and
establish the mapping in accordance with the empirical recipes, the material property and its variation trend.

15. A computer-readable storage medium having instructions stored thereon that, when executed by a processor, cause the processor to perform the method of any one of claims 1-8.
